# EUROPÄISCHE PATENTANMELDUNG

(11) **EP 3 315 483 A1**
(43) Veröffentlichungstag der Anmeldung: **02.05.2018**
(21) Anmeldenummer: 16195605.7
(22) Anmeldetag: 25.10.2016
(51) Int. Cl.: C07C 2/84, C07C 11/04

(54) **VERFAHREN ZUR GEWINNUNG VON ETHYLEN AUS METHAN**

(71) Anmelder: Linde Aktiengesellschaft, 80331 München (DE)
(72) Erfinder: FRITZ, Helmut, 81375 München (DE); OBERLE, David, 81476 München (DE); MÜNDL, Florian, 83624 Otterfing (DE)
(74) Vertreter: m patent group

(57) **Zusammenfassung**

Die Erfindung betrifft ein Verfahren zur Gewinnung von Ethylen, bei dem ein Methan enthaltender Reaktionseinsatz unter Erhalt eines Prozessgases einer oxidativen Kopplung von Methan unterworfen wird und zumindest ein Teil des gebildeten Prozessgases einer Abkühlung unterworfen wird. Es ist vorgesehen, dass die Abkühlung einen ersten Abkühlschritt von einem ersten Temperaturniveau auf ein zweites Temperaturniveau und einen zweiten Abkühlschritt von einem dritten auf ein viertes Temperaturniveau umfasst, wobei das erste Temperaturniveau oberhalb des zweiten Temperaturniveaus und das dritte Temperaturniveau oberhalb des vierten Temperaturniveaus liegt und das zweite Temperaturniveau dem dritten Temperaturniveau entspricht oder oberhalb des dritten Temperaturniveaus liegt, dass in dem ersten Abkühlschritt Wärme auf Kesselspeisewasser auf einem Kesselspeisewassertemperaturniveau und einem Kesselspeisewasserdruckniveau übertragen und dadurch Sattdampf auf einem Sattdampfdruckniveau und einem Sattdampftemperaturniveau gebildet wird, und dass in dem zweiten Abkühlschritt Wärme auf zumindest einen Teil des Sattdampfs übertragen und dadurch Heißdampf auf einem Heißdampfdruckniveau und einem Heißdampftemperaturniveau gebildet wird. Eine entsprechende Anlage (100) ist ebenfalls Gegenstand der Erfindung.

## Beschreibung

Die Erfindung betrifft ein Verfahren zur Gewinnung von Ethylen aus Methan und eine entsprechende Anlage gemäß den Oberbegriffen der unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet.

Die Reaktionsbedingungen bei der oxidativen Kopplung von Methan umfassen klassischerweise eine Temperatur von 500 bis 900 °C und einen Druck von 1 bis 10 bar sowie hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden; der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid. Ein bei der oxidativen Kopplung von Methan erhaltenes Gasgemisch, nachfolgend als "Prozessgas" bezeichnet, enthält daher neben dem in vergleichsweise geringer Menge enthaltenen Zielprodukt Ethylen und ggf. anderen Olefinen auch Nebenprodukte und große Mengen in der Reaktion nicht umgesetzten Methans.

Für einen möglichst effektiven Reaktionsumsatz muss das Prozessgas nach Durchlaufen des Reaktors einen möglichst schnellen Abkühlvorgang erfahren, da ansonsten die erwähnten unerwünschten Nebenreaktionen begünstigt würden. Die aus dem Stand der Technik hierzu bekannten Lösungen sind jedoch aus unterschiedlichen Gründen, die nachfolgend noch erläutert werden, nicht optimal.

Es besteht daher der Bedarf nach verbesserten Verfahren zur Gewinnung von Ethylen aus Methan unter Einsatz der oxidativen Kopplung von Methan, insbesondere im Hinblick auf die Abkühlung des Prozessgases.

### Offenbarung der Erfindung

Vor diesem Hintergrund schlägt die vorliegende Erfindung ein Verfahren und eine Anlage zur Gewinnung von Ethylen aus Methan mit den Merkmalen der unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Bei der oxidativen Kopplung von Methan können Reaktoren eingesetzt werden, in denen einer katalytischen Zone eine nichtkatalytische Zone nachgeschaltet ist. Das die katalytische Zone verlassende Prozessgas strömt dabei direkt in die nichtkatalytische Zone ab, wo es zunächst noch den hohen Temperaturen ausgesetzt ist, die in der katalytischen Zone herrschen. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Ist hier davon die Rede, dass ein methanreicher Reaktionseinsatz "unter Erhalt eines Prozessgases (zumindest) einer oxidativen Kopplung von Methan unterworfen wird", soll damit zum Ausdruck gebracht werden, dass hierbei nicht ausschließlich die eingangs erwähnten eigentlichen Reaktionsschritte bei der oxidativen Kopplung von Methan zum Einsatz kommen müssen. Vielmehr können auch weitere Reaktionsschritte, insbesondere jene, die beim postkatalytischen Dampfspalten auftreten können, umfasst sein. Entsprechendes gilt auch, wenn hier von einem Reaktor die Rede ist, der "(zumindest) zur oxidativen Kopplung von Methan eingerichtet" ist.

Bezüglich den beim Dampfspalten herrschenden Reaktionsbedingungen sei auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste ein. Entsprechendes gilt für Temperaturniveaus. Bei dem hier in bar angegebenen Druckniveaus handelt es sich um Absolutdrücke.

### Vorteile der Erfindung

Wie bereits erwähnt, muss das Prozessgas nach dem Durchlaufen eines Reaktors zur oxidativen Kopplung von Methan für einen möglichst effektiven Reaktionsumsatz einen möglichst schnellen Abkühlvorgang erfahren, da ansonsten die erwähnten unerwünschten Nebenreaktionen begünstigt würden.

Das zwischen 900 und 800 °C heiße Prozessgas muss dabei sehr schnell auf Temperaturen um 650 °C oder darunter (insbesondere weniger als 670 °C) abgekühlt werden um die unerwünschte Weiterreaktion der Zielproduktkomponenten (Ethylen und anderer Mono- und Diolefine) zu unterbinden. Dies kann im indirektem Wärmeaustausch mit siedendem Wasser erfolgen, d.h. unter Erzeugung von gesättigtem Dampf bei Drücken zwischen 40 und 120 bar. Dem Prozessgas werden dabei sehr hohe Energiemengen entzogen, die es sinnvoll zu verwenden gilt.

Nachdem die Weiterverarbeitung des Prozessgases hohe Wellenleistungen für Produktgas- und Kältemittelverdichter benötigt, ist es besonders vorteilhaft, den erzeugten Dampf für Dampfturbinen für den Verdichterantrieb zu nutzen. Hierfür muss der Sattdampf aber auf Temperaturen von bis zu 525 °C überhitzt werden. Dies kann in befeuerten Dampfüberhitzeröfen oder unter Verwendung entsprechend heißer Medien wie beispielsweise dem Turbinenabgas einer Gasturbine erfolgen. In beiden Fällen ist jedoch eine separate ggf. zusätzliche Wärmequelle erforderlich und damit ein zusätzlichen Einsatz von Primärenergie wie beispielsweise Erdgas oder Heizgas zur Unterfeuerung eines Dampfüberhitzerofens oder den Betrieb einer Gasturbine.

Im Rahmen der vorliegenden Erfindung wird der zusätzliche Energieaufwand für die Überhitzung des Sattdampfes nun dadurch umgangen, dass der Sattdampf durch das heiße Prozessgas aus der oxidativen Kopplung von Methan, welches zuvor aber bereits durch für eine Sattdampferzeugung genutzt wurde, in einem der Sattdampferzeugung nachgeschaltetem Apparat auf die gewünschte Temperatur überhitzt wird. Abhängig vom gewünschten Dampfdruck des überhitzten Sattdampfes liegt die erforderliche Überhitzungstemperatur zwischen 550 und 400°C.

Diese Anordnung der Verfahrensschritte (also eine Sattdampferzeugung bei höherer Temperatur des Prozessgases als die Sattdampfüberhitzung) ist kontraintuitiv, da normalerweise die Überhitzung mit dem heißen Medium (hier dem Prozessgas aus der oxidativen Kopplung von Methan) bei höherer Temperatur und nicht, wie erfindungsgemäß, bei niedrigeren Temperatur erfolgt als die Verdampfung.

Die Ausgestaltung der jeweils verwendeten Wärmeübertrager genau aufeinander abzustimmen. Der Prozessgastemperatur zwischen den beiden Wärmeübertragern kommt dabei die entscheidende Bedeutung zu. Sie muss so hoch gewählt werden, dass der Energiegehalt im Prozessgas noch ausreicht und bei ausreichend hoher Temperatur vorliegt, damit die erzeugte Sattdampfmenge noch auf die gewünschte Überhitzungstemperatur aufgeheizt werden kann. Ist eine entsprechende Abstimmung erfolgt, kann im Rahmen der vorliegenden Erfindung jedoch eine besonders effiziente und auf die Temperaturen des Prozessgases abgestimmte Wärmenutzung erfolgen.

Grundsätzlich gilt, dass im Rahmen der vorliegenden Erfindung die erwähnte Zwischentemperatur mit dem Sattdampfdruck abgesenkt werden kann (aber nicht muss), ebenso die Temperatur des Prozessgases beim Verlassen des Überhitzers, womit die Energiemenge die dem Prozessgas entzogen werden kann, steigt. Hierin besteht ein wesentlicher Vorteil, der im Rahmen der vorliegenden Erfindung erzielt werden kann.

Die vorliegende Erfindung schlägt ein Verfahren zur Gewinnung von Ethylen vor, bei dem ein Methan enthaltender Reaktionseinsatz unter Erhalt eines Prozessgases einer oxidativen Kopplung von Methan unterworfen wird und zumindest ein Teil des gebildeten Prozessgases einer Abkühlung unterworfen wird. Wie erwähnt, sind entsprechende Verfahren grundsätzlich bekannt, so dass hierzu erneut auf Fachliteratur verwiesen werden kann.

Im Rahmen der vorliegenden Erfindung ist vorgesehen, dass die Abkühlung einen ersten Abkühlschritt von einem ersten Temperaturniveau auf ein zweites Temperaturniveau und einen zweiten Abkühlschritt von einem dritten auf ein viertes Temperaturniveau umfasst, wobei das erste Temperaturniveau oberhalb des zweiten Temperaturniveaus und das dritte Temperaturniveau oberhalb des vierten Temperaturniveaus liegt und das zweite Temperaturniveau dem dritten Temperaturniveau entspricht oder oberhalb des dritten Temperaturniveaus liegt. Wie bereits angesprochen, umfasst die vorliegende Erfindung zunächst die Erzeugung von Sattdampf und anschließend die Überhitzung des Sattdampfs. Es ist also vorgesehen, dass in dem ersten Abkühlschritt Wärme auf Kesselspeisewasser auf einem Kesselspeisewassertemperaturniveau und einem Kesselspeisewasserdruckniveau übertragen und dadurch Sattdampf auf einem Sattdampfdruckniveau und einem Sattdampftemperaturniveau gebildet wird, und dass in dem zweiten Abkühlschritt Wärme auf zumindest einen Teil des Sattdampfs übertragen und dadurch Heißdampf auf einem Heißdampfdruckniveau und einem Heißdampftemperaturniveau gebildet wird. Weitere Details und Vorteile wurden bereits erläutert. Unter "Heißdampf" wird dabei hier überhitzter Sattdampf verstanden.

Im Rahmen der vorliegenden Erfindung kann insbesondere vorgesehen sein, dass für den zweiten Abkühlschritt ein oder mehrere Wärmetauscher mit einer oder mehreren internen und/oder externen Bypassleitungen für das Prozessgas verwendet werden. Durch entsprechende Bypassleitungen kann einerseits ein Umfang, in dem das Prozessgas abgekühlt wird, und andererseits der Umfang, in dem die Überhitzung des Sattdampfs vorgenommen wird, eingestellt werden. Auf diese Weise ist eine vorteilhafte Regelung entsprechender Temperaturen und, im Falle des Dampfs, auch der damit verbundenen Drücke möglich.

Im Rahmen der vorliegenden Erfindung kann insbesondere auch vorgesehen sein, dass für den zweiten Abkühlschritt zwei seriell angeordnete Wärmetauscher verwendet werden. Auf diese Weise lässt sich eine besonders effiziente Wärmeübertragung sicherstellen. Vorteilhafterweise kann in diesem Fall das Heißdampftemperaturniveau stromab zumindest eines der Wärmetauscher durch Abspritzung eingestellt werden, also durch eine Wassereinspritzung in den Dampfweg, die das Dampfvolumen bzw. den Dampfdruck erhöht und die Temperatur verringert. Auf diese Weise kann dem Prozessgas eine höhere Wärmemenge entzogen werden.

Wie erwähnt, sind im Rahmen der vorliegenden Erfindung die Abkühlung in dem ersten und zweiten Abkühlschritt und die Ausgestaltung der jeweils verwendeten Wärmetauscher genau aufeinander abzustimmen. Der Prozessgastemperatur zwischen dem ersten und dem zweiten Abkühlschritt kommt dabei, wie erwähnt, die entscheidende Bedeutung zu.

Vorteilhafterweise liegt dabei das erste Temperaturniveau bei 800 bis 900 °C, insbesondere bei 830 bis 900 °C und/oder das zweite Temperaturniveau bei 300 bis 700 °C, insbesondere bei 450 bis 650 °C, weiter ins besondere bei 480 bis 580 °C und/oder das dritte Temperaturniveau bei bei 300 bis 700 °C, insbesondere bei 450 bis 650 °C, weiter insbesondere bei 480 bis 580 °C und/ oder das vierte Temperaturniveau bei 300 bis 400 °C, insbesondere bei 350 bis 450 °C. Diese Temperaturniveaus können im Rahmen der Erfindung insbesondere dann eingesetzt werden, wenn das Kesselspeisewasserdruckniveau bei 20 bis 150 bar, insbesondere bei 45 bis 120 bar und/oder das Kesselspeisewassertemperaturniveau bei 50 bis 150 °C, insbesondere bei 80 bis 100 °C liegt, und/oder wenn das Heißdampfdruckniveau bei 20 bis 120 °C, insbesondere bei 40 bis 120 °C und/oder das Heißdam pftemperaturniveau bei 400 bis 550 °C, insbesondere bei 450 bis 525 °C liegt.

Im Rahmen der vorliegenden Erfindung können auch noch weitere Abkühlschritte vorteilhafterweise vorgesehen sein. Hierbei kann die Abkühlung einen dritten Abkühlschritt von einem fünften Temperaturniveau auf ein sechstes Temperaturniveau umfassen, wobei das fünfte Temperaturniveau dem vierten Temperaturniveau entspricht oder oberhalb des vierten Temperaturniveaus liegt. In dem dritten Abkühlschritt kann insbesondere Wärme auf weiteren Sattdampf übertragen und Heißdampf auf einem weiteren Druckniveau und einem weiteren Temperaturniveau erzeugt werden. Auf diese Weise kann nach dem zweiten Abkühlschritt vorhandene Restwärme in besonders vorteilhafter Weise genutzt werden.

Es kann im Rahmen der vorliegenden Erfindung auch vorgesehen sein, dass die Abkühlung einen weiteren Abkühlschritt umfasst, in dem das Kesselspeisewasser auf das Kesselspeisewassertemperaturniveau erwärmt wird. Auf diese Weise kann Restwärme weiter genutzt werden. Dies gilt auch für eine weitere Verfahrensvariante, bei der die Abkühlung einen weiteren Abkühlschritt umfasst, in dem der Reaktionseinsatz erwärmt wird.

Eine Anlage zur Gewinnung von Ethylen, mit einem Reaktor, der dazu eingerichtet ist, einen Methan enthaltenden Reaktionseinsatz unter Erhalt eines Prozessgases einer oxidativen Kopplung von Methan zu unterwerfen und mit Mitteln, die dazu eingerichtet sind, zumindest ein Teil des gebildeten Prozessgases einer Abkühlung zu unterwerfen, ist ebenfalls Gegenstand der vorliegenden Erfindung.

Erfindungsgemäß sind für die Abkühlung wenigstens ein erster Wärmetauscher, der dazu eingerichtet ist, einen ersten Abkühlschritt von einem ersten Temperaturniveau auf ein zweites Temperaturniveau vorzunehmen, und wenigstens ein zweiter Wärmetauscher, der dazu eingerichtet ist, einen zweiten Abkühlschritt von einem dritten auf ein viertes Temperaturniveau vorzunehmen, vorgesehen, wobei das erste Temperaturniveau oberhalb des zweiten Temperaturniveaus und das dritte Temperaturniveau oberhalb des vierten Temperaturniveaus liegt und das zweite Temperaturniveau dem dritten Temperaturniveau entspricht oder oberhalb des dritten Temperaturniveaus liegt.

Ferner sind Mittel vorgesehen, die dazu eingerichtet sind, in dem wenigstens einen ersten Wärmetauscher Wärme auf Kesselspeisewasser auf einem Kesselspeisewassertemperaturniveau und einem Kesselspeisewasserdruckniveau zu übertragen und dadurch Sattdampf auf einem Sattdampfdruckniveau und einem Sattdampftemperaturniveau zu bilden, Schließlich sind Mittel vorgesehen, die dazu eingerichtet sind, in dem zweiten Abkühlschritt Wärme auf zumindest einen Teil des Sattdampfs zu übertragen und dadurch Heißdampf auf einem Heißdampfdruckniveau und einem Heißdampftemperaturniveau zu bilden.

Eine entsprechende Anlage ist insbesondere zur Ausführung eines Verfahrens eingerichtet, wie es zuvor erläutert wurde. Auf die entsprechenden Erläuterungen wird daher ausdrücklich verwiesen.

Die Erfindung wird in bevorzugten Ausgestaltungen unter Bezugnahme auf die beigefügten Zeichnungen näher erläutert.

### Kurze Beschreibung der Zeichnung

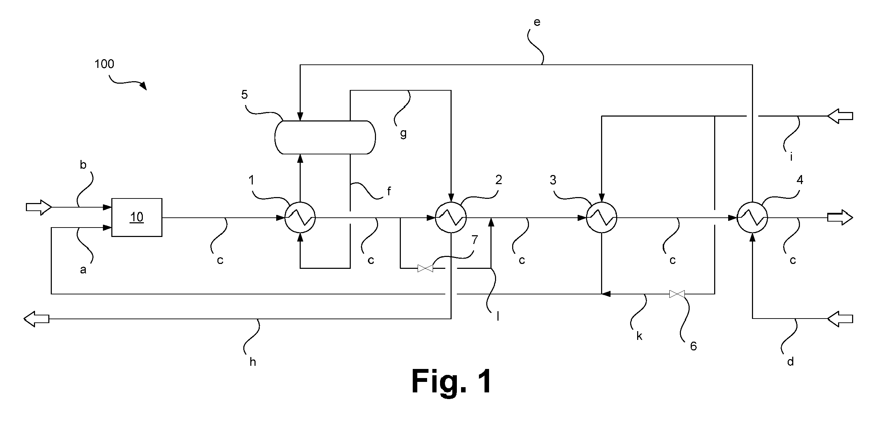

Figur 1 veranschaulicht eine Anlage gemäß einer Ausführungsform der Erfindung in Form eines schematischen Prozessflussdiagramms.

### Ausführliche Beschreibung der Zeichnung

In Figur 1 ist eine Anlage gemäß einer besonders bevorzugten Ausführungsform der Erfindung in Form eines schematischen Prozessflussdiagramms veranschaulicht und insgesamt mit 100 bezeichnet. Die Erläuterungen bezüglich Anlage 100 betreffen ein Verfahren gemäß einer besonders bevorzugten Ausführungsform der Erfindung in gleicher Weise, so dass die veranschaulichten Anlagenkomponenten auch als entsprechende Verfahrensschritte aufgefasst werden können.

In der Anlage 100 wird im dargestellten Beispiel ein Methan enthaltender Reaktionseinsatz in Form eines Stoffstroms a, der auf ein für die Reaktion geeignetes Temperaturniveau erwärmt wurde, einem Reaktor 10 zugeführt, der zur oxidativen Kopplung von Methan eingerichtet ist. Dem Reaktor 10 wird ferner ein sauerstoffreicher Reaktionseinsatz, hier in Form eines Stoffstroms b veranschaulicht, zugeführt. Es sei betont, dass in einer entsprechenden Anlage 100 auch mehrere Reaktoren 10 vorhanden sein können, denen dann jeweils Reaktionseinsätze zugeführt werden. Auch die Verwendung weiterer Reaktionseinsätze ist möglich. Der Reaktor 10 ist stark vereinfacht veranschaulicht. Er kann insbesondere auch eine Stufe zur Durchführung eines postkatalytischen Dampfspaltens umfassen. Insbesondere dieser können weitere Gase oder Gasgemische zugeführt werden. Unter Verwendung des Reaktors 10 wird ein Prozessgas in Form eines Stoffstroms c erhalten.

Das Prozessgas, das auf einem Temperaturniveau vorliegt, das höher ist, das das, auf dem der Reaktionseinsatz dem Reaktor 10 zugeführt wurde, wird, wie in Form des Stoffstroms c veranschaulicht, nun zumindest teilweise abgekühlt. Hierbei sind nacheinander mehrere Abkühlschritte vorgesehen, von denen nachfolgend zunächst ein erster und ein zweiter Abkühlschritt erläutert werden.

In dem in Figur 1 dargestellten Beispiel wird der erste Abkühlschritt unter Verwendung eines ersten Wärmetauschers 1 und der zweite Abkühlschritt unter Verwendung eines zweiten Wärmetauschers 2 vorgenommen. Es können auch jeweils mehrere erste Wärmetauscher 1 und/oder zweite Wärmetauscher 2 vorgesehen sein, wie bereits zuvor erläutert. Lediglich der Einfachheit halber ist nachfolgend jeweils von "einem" ersten Wärmetauscher 1 und "einem" zweiten Wärmetauscher 2 die Rede.

Stromauf des ersten Wärmetauschers 1 liegt das Prozessgas auf einem ersten Temperaturniveau, stromab des ersten Wärmetauschers 1 auf einem zweiten Temperaturniveau vor. Stromauf des zweiten Wärmetauschers 2 liegt das Prozessgas auf einem dritten Temperaturniveau, stromab des ersten Wärmetauschers 2 auf einem vierten Temperaturniveau vor. Das erste Temperaturniveau liegt oberhalb des zweiten Temperaturniveaus und das dritte Temperaturniveau liegt oberhalb des vierten Temperaturniveaus. Das zweite Temperaturniveau kann dem dritten Temperaturniveau entsprechen oder oberhalb des dritten Temperaturniveaus liegen. Letzteres kann insbesondere dann der Fall sein, wenn zwischen dem ersten Wärmetauscher 1 und dem zweiten Wärmetauscher 2 weitere Wärmetauscher angeordnet sind oder eine anderweitige Abkühlung erfolgt. Der erste Wärmetauscher 1 und der zweite Wärmetauscher 2 können gleich oder unterschiedlich ausgebildet sein.

Kesselspeisewasser, das zuvor auf ein Temperaturniveau erwärmt wurde, das hier auch als "Kesselspeisewassertemperaturniveau" bezeichnet wird, wird einer Dampftrommel 5 zugeführt, wie hier Form eines Stoffstroms e veranschaulicht. Wiederum können mehrere Dampftrommeln 5 vorhanden sein und es wird lediglich der Einfachheit halber von "einer" Dampftrommel 5 gesprochen. In dem ersten Abkühlschritt wird mittels des ersten Wärmetauschers 1 Wärme in die Dampftrommel 5 eingeleitet. Hierzu wird der Dampftrommel kontinuierlich Wasser entnommen und in Form eines Stoffstroms f durch den Wärmetauscher 1 geführt. Eine Wärmeübertragung kann jedoch auch auf andere Weise erfolgen. In der Dampftrommel 5 wird Sattdampf erzeugt, der auf einem hier als "Sattdampfdruckniveau" bezeichneten Druck- und einem hier als "Sattdampftemperaturniveau" bezeichneten Temperaturniveau vorliegt. Es wird also in dem ersten Abkühlschritt Wärme auf Kesselspeisewasser auf einem Kesselspeisewassertemperaturniveau und einem Kesselspeisewasserdruckniveau übertragen und dadurch Sattdampf auf einem Sattdampfdruckniveau und einem Sattdampftemperaturniveau gebildet.

Der Sattdampf wird in Form eines Stoffstroms g aus der Dampftrommel 5 abgezogen und durch den zweiten Wärmetauscher 2 geführt, der in dem zuvor erläuterten zweiten Abkühlschritt des Prozessgases eingesetzt wird. In dem zweiten Wärmetauscher 2 wird der Sattdampf auf ein hier als "Heißdampfdruckniveau" bezeichnetes Temperaturniveau überhitzt. Der Sattdampf kann auf diesem Heißdampfdruckniveau und einem hier als "Heißdampfdruckniveau" bezeichneten Druckniveau in Form eines Dampfstroms h Verbrauchern, beispielsweise Dampfturbinen, zugeführt werden. Es wird also in dem zweiten Abkühlschritt Wärme auf zumindest einen Teil des Sattdampfs übertragen und dadurch Heißdampf auf einem Heißdampfdruckniveau und einem Heißdampftemperaturniveau gebildet.

In Figur 4 sind zwei weitere Abkühlschritte veranschaulicht, die unter Verwendung zweier weiterer Wärmetauscher 3 und 4 (oder, wie oben, jeweils mehrere entsprechende Wärmetauscher) durchgeführt werden. In dem Wärmetauscher 4 bzw. dem entsprechenden Abkühlschritt wird Wärme des Prozessgases zum Erwärmen von Kesselspeisewasser auf das erwähnte Kesselspeisewassertemperaturniveau, auf dem dieses der Dampftrommel 5 zugeführt wird, verwendet.

Der Wärmetauscher 3 bzw. der entsprechende Abkühlschritt wird hingegen nicht zur Erzeugung von Dampf oder zur Erwärmung von Kesselspeisewasser, sondern zum Erwärmen des Methan enthaltenden Reaktionseinsatzes verwendet. Der Reaktionseinsatz, der direkt stromauf des Reaktors 10, wie bereits erwähnt, in Form eines Stoffstroms a veranschaulicht ist, ist in Figur 1 anfänglich, d.h. vor der Erwärmung in dem Wärmetauscher 3, noch in Form eines Stoffstroms i dargestellt. Der Reaktionseinsatz wird hier auf ein geeignetes Temperaturniveau erwärmt. Das Temperaturniveau kann durch Zuspeisen von nicht entsprechend vorgewärmtem Reaktionseinsatz, also über einen Bypass des Wärmetauschers 3, eingestellt werden, wie hier in Form eines Stoffstroms k mit einem Ventil 6 veranschaulicht.

Auch der zweite Wärmetauscher 2 kann mittels eines Bypasses, wie hier in Form eines Stoffstroms I mit einem Ventil 7 veranschaulicht, umgangen werden. Auf diese Weise ist es möglich, einen Umfang der Abkühlung des gesamten Prozessgases und einen Umfang der Überhitzung des Sattdampfs einzustellen.

## Patentansprüche

1. Verfahren zur Gewinnung von Ethylen, bei dem ein Methan enthaltender Reaktionseinsatz unter Erhalt eines Prozessgases einer oxidativen Kopplung von Methan unterworfen wird und zumindest ein Teil des gebildeten Prozessgases einer Abkühlung unterworfen wird, **dadurch gekennzeichnet, dass** die Abkühlung einen ersten Abkühlschritt von einem ersten Temperaturniveau auf ein zweites Temperaturniveau und einen zweiten Abkühlschritt von einem dritten auf ein viertes Temperaturniveau umfasst, wobei das erste Temperaturniveau oberhalb des zweiten Temperaturniveaus und das dritte Temperaturniveau oberhalb des vierten Temperaturniveaus liegt und das zweite Temperaturniveau dem dritten Temperaturniveau entspricht oder oberhalb des dritten Temperaturniveaus liegt, dass in dem ersten Abkühlschritt Wärme auf Kesselspeisewasser auf einem Kesselspeisewassertemperaturniveau und einem Kesselspeisewasserdruckniveau übertragen und dadurch Sattdampf auf einem Sattdampfdruckniveau und einem Sattdampftemperaturniveau gebildet wird, und dass in dem zweiten Abkühlschritt Wärme auf zumindest einen Teil des Sattdampfs übertragen und dadurch Heißdampf auf einem Heißdampfdruckniveau und einem Heißdampftemperaturniveau gebildet wird.

2. Verfahren nach Anspruch 1, bei dem für den zweiten Abkühlschritt ein oder mehrere Wärmetauscher (2) mit einer oder mehreren internen und/oder externen Bypassleitungen für das Prozessgas verwendet werden.

3. Verfahren nach Anspruch 1 oder 2, bei dem für den zweiten Abkühlschritt zwei seriell angeordnete Wärmetauscher (2) verwendet werden.

4. Verfahren nach Anspruch 3, bei dem das Heißdampftemperaturniveau stromab zumindest eines der Wärmetauscher (2) durch Abspritzung eingestellt wird.

5. Verfahren nach einem der vorstehenden Ansprüche, bei dem das erste Temperaturniveau bei 800 bis 900 °C liegt und/oder das zweite Temperaturniveau bei 300 bis 700 °C liegt und/oder das dritte Temper aturniveau bei 300 bis 700 °C liegt und/oder das vierte Temperaturniveau 300 bis 400 °C liegt.

6. Verfahren nach einem der vorstehenden Ansprüche, bei dem das Kesselspeisewasserdruckniveau bei 20 bis 150 bar liegt und/oder das Kesselspeisewassertemperaturniveau bei 50 bis 150 °C liegt.

7. Verfahren nach einem der vorstehenden Ansprüche, bei dem das Heißdampfdruckniveau bei 20 bis 120 °C liegt und/od er das Heißdampftemperaturniveau bei 400 bis 550 °C liegt.

8. Verfahren nach einem der vorstehenden Ansprüche, bei dem die Abkühlung einen dritten Abkühlschritt von einem fünften Temperaturniveau auf ein sechstes Temperaturniveau umfasst, wobei das fünfte Temperaturniveau dem vierten Temperaturniveau entspricht oder oberhalb des vierten Temperaturniveaus liegt.

9. Verfahren nach Anspruch 8, bei dem in dem dritten Abkühlschritt Wärme auf weiteren Sattdampf übertragen und Heißdampf auf einem weiteren Druckniveau und einem weiteren Temperaturniveau erzeugt wird.

10. Verfahren nach einem der vorstehenden Ansprüche, bei dem die Abkühlung einen weiteren Abkühlschritt umfasst, in dem das Kesselspeisewasser auf das Kesselspeisewassertemperaturniveau erwärmt wird.

11. Verfahren nach einem der vorstehenden Ansprüche, bei dem die Abkühlung einen weiteren Abkühlschritt umfasst, in dem der Reaktionseinsatz erwärmt wird.

12. Anlage (100) zur Gewinnung von Ethylen, mit einem Reaktor (10), der dazu eingerichtet ist, einen Methan enthaltenden Reaktionseinsatz unter Erhalt eines Prozessgases einer oxidativen Kopplung von Methan zu unterwerfen und mit Mitteln, die dazu eingerichtet sind, zumindest ein Teil des gebildeten Prozessgases einer Abkühlung zu unterwerfen, **dadurch gekennzeichnet, dass** für die Abkühlung wenigstens ein erster Wärmetauscher (1), der dazu eingerichtet ist, einen ersten Abkühlschritt von einem ersten Temperaturniveau auf ein zweites Temperaturniveau vorzunehmen, und wenigstens ein zweiter Wärmetauscher (2), der dazu eingerichtet ist, einen zweiten Abkühlschritt von einem dritten auf ein viertes Temperaturniveau vorzunehmen, vorgesehen sind, wobei das erste Temperaturniveau oberhalb des zweiten Temperaturniveaus und das dritte Temperaturniveau oberhalb des vierten Temperaturniveaus liegt und das zweite Temperaturniveau dem dritten Temperaturniveau entspricht oder oberhalb des dritten Temperaturniveaus liegt, und dass Mittel vorgesehen sind, die dazu eingerichtet sind, in dem wenigstens einen ersten Wärmetauscher (1) Wärme auf Kesselspeisewasser auf einem Kesselspeisewassertemperaturniveau und einem Kesselspeisewasserdruckniveau zu übertragen und dadurch Sattdampf auf einem Sattdampfdruckniveau und einem Sattdampftemperaturniveau zu bilden, und dass Mittel vorgesehen sind, die dazu eingerichtet sind, in dem zweiten Abkühlschritt Wärme auf zumindest einen Teil des Sattdampfs zu übertragen und dadurch Heißdampf auf einem Heißdampfdruckniveau und einem Heißdampftemperaturniveau zu bilden.
